# EUROPÄISCHE PATENTSCHRIFT

(11) **EP 3 586 783 B1**
(45) Veröffentlichungstag und Bekanntmachung des Hinweises auf die Patenterteilung: **27.01.2021**
(21) Anmeldenummer: 19178340.6
(22) Anmeldetag: 05.06.2019
(51) Int. Cl.: A61B 34/30, A61B 17/34, A61B 17/00

(54) **TROKARHALTERUNG**
TROCAR HOLDER
SUPPORT DE TROCART

(30) Priorität: 27.06.2018 DE 102018115435
(43) Veröffentlichungstag der Anmeldung: 01.01.2020
(73) Patentinhaber: avateramedical GmbH, 07745 Jena (DE)
(72) Erfinder: Keim, Tobias, 07343 Wurzbach (DE); Kreißig, Albrecht, 70599 Stuttgart (DE); Pudewills, Leif, 73728 Esslingen (DE); Waibel, Tobias, 73760 Ostfildern (DE)
(74) Vertreter: Gleim Petri Oehmke Patent- und Rechtsanwaltspartnerschaft mbB

(56) Entgegenhaltungen:
- EP-A2- 1 925 260
- WO-A1-00/18306
- WO-A1-03/092518
- WO-A1-2013/075205
- DE-A1-102013 002 813
- DE-A1-102016 111 737
- US-A1- 2010 292 724
- US-A1- 2018 168 746

## Beschreibung

### Gebiet der Erfindung

Die Erfindung betrifft eine Trokarhalterung für einen Manipulator eines robotischen Operationssystems. Eine solche Trokarhalterung umfasst ein Basiselement zur Befestigung der Trokarhalterung am Manipulator. Außerdem umfasst sie ein Klammerelement mit zwei Schenkeln, welches über einen Kopplungsmechanismus mit dem Basiselement auswechselbar verbunden ist. Die beiden Schenkel sind relativ zueinander beweglich und über einen Rücken miteinander verbunden, sie sind mit freien Spitzen ausgebildet. Schließlich umfasst die Trokarhalterung einen Klammermechanismus zum Offnen und Schließen der Schenkel und damit der Klammer. Darüberhinaus umfasst eine solche Trokarhalterung ein Aufnahmelement für einen Trokar, das als Achse ausgebildet ist. Das Aufnahmelement umfasst eine durchgehende Öffnung zur Durchführung des Trokars senkrecht durch die Achse. Im Bereich der Spitzen jedes der beiden Schenkel, ist ein Radiaxlager ausgebildet. Das Aufnahmeelement ist bei geschlossenen Schenkeln von den Radiaxlagern an einer axialen Bewegung gehindert.

Für eine steigende Anzahl minimalinvasiver Operationen insbesondere an schwer zugänglichen Stellen werden heutzutage robotische Operationssysteme verwendet. Ein solches Operationssystem umfasst in der Regel eine Anzahl von Manipulatorarmen, die an ihrem einen Ende mit einer tragenden Vorrichtung gekoppelt sind und an ihrem anderen Ende eine Instrumententrägervorrichtung aufweisen. Zusammen bilden sie einen Manipulator. Die Steuerung erfolgt über ein Bedienpult, welches sich nicht unbedingt in unmittelbarer Nähe zu den Manipulatoren befinden muss.

Über das Bedienpult bedient der Operateur, d.h. der Chirurg, meist mehrere robotisch gesteuerte Arme, welche die Instrumente halten, um einzelne Schritte einer Operation durchzuführen. Über das Bedienpult oder durch entsprechende Bewegungen über einen geeigneten Koppelmechanismus, der Bewegungen der Hände des Chirurgen in Bewegungen des entsprechenden Instruments umsetzt, steuert der Chirurg die Manipulatorarme des Systems. Die Manipulatoren sind vielgliedrige Systeme, bei denen die einzelnen Glieder über Gelenke miteinander verbunden sind. Ein Teil der Glieder dient der Positionierung des Manipulators relativ zu anderen Manipulatoren desselben robotischen Systems, so dass sich die Manipulatoren nicht gegenseitig behindern.

Dieser Teil der Glieder wird auch als Stellvorrichtung bezeichnet, die beschriebene Positionierung erfolgt vor der Operation und wird daher auch als Vorpositionierung bezeichnet.

Ein anderer Teil der Glieder dient der Positionierung und Führung des Werkzeuges am Objekt, also beispielsweise der Positionierung eines Operationsinstruments oder eines Endoskops im Bereich einer Öffnung in der Gewebedecke eines Patienten. Dieser Teil der Glieder wird auch als Instrumententrägervorrichtung bezeichnet. Die Glieder eines Manipulators sind im kinematischen Sinne als offene kinematische Kette miteinander verbunden, da am letzten Glied kein weiteres Gelenk sitzt, sondern das Werkzeug. Alle anderen Glieder sind nach Art einer Kette über Gelenke miteinander verbunden.

Zur Durchführung minimalinvasiver Eingriffe werden sogenannte Trokare gesetzt, dabei handelt es sich um medizinische Instrumente, mit denen zunächst ein Zugang in den Körper geschaffen wird, beispielsweise in den Bauchraum. Dieser Zugang wird dann mittels des Trokars, der die Form eines Tubus aufweist, offengehalten. Der Tubus dient zur Einführung von chirurgischen Instrumenten in den Körper, aber auch von endoskopischen Kameras. Während der Operation sollte der Trokar seine Position nicht verändern, d.h. stabil in einer vorgegebenen Position angeordnet bleiben, es sei denn, es ist etwas anderes gefordert.

Auch bei robotischen Operationssystemen werden Trokare gesetzt und während der Operation in Position gehalten. Die Instrumententrägervorrichtungen sind dafür in der Regel mit einer Trokarhalterung ausgestattet, durch welche das Instrument dann in den Körper geführt werden kann. Nach einer Vorpositionierung der Trokarhalterungen an den verschiedenen Manipulatoren des robotischen Operationssystems können die Trokare und anschließend die Instrumente eingeführt werden. Dabei ist darauf zu achten, dass die Belastung des Gewebes an den Stellen, wo die Trokare eingeführt werden, möglichst geringgehalten wird. Darüber hinaus ist der Bereich der Trokarhalterungen und der Trokare steril zu halten. Trokare können als Einweg- oder Mehrwegkomponenten ausgestaltet sein, gleiches gilt für die Trokarhalterungen. Wichtig ist, dass die Trokarhalterungen ebenfalls gewechselt werden können, um beispielsweise eine neue Operation vorzubereiten oder einen anderen Trokar anzusetzen.

Üblicherweise erfolgt die Befestigung des Klammerelements über das Basiselement am Manipulator in der Weise, dass eine Relativbewegung zwischen beiden im montierten bzw. verbundenen Zustand ausgeschlossen ist, da über das Basiselement gegebenenfalls auch ein elektrischer Kontrakt mit einer Instrumentenführung bzw. mit dem Manipulator hergestellt wird, der beispielsweise ein elektrisches Öffnen und Schließen der Klammer erlaubt. In diesem Fall wird zur Einhaltung des Gebots der Verwendung steriler Instrumente eine diese Bedingung sichernde Sterilisationsfolie oder ein ähnliches Mittel in die Klammer, die dann den Trokar hält, eingelegt. Da die Trokarhalterung selbst nicht steril ist und dennoch in relativ großer Nähe zum Patienten geführt wird, besteht hier in manchen Fällen ein erhöhtes Infektionsrisiko.

### Stand der Technik

Im Zusammenhang mit robotischen Operationssystemen sind im Stand der Technik verschiedene Trokarhalterungen, mit denen allgemein der Trokar relativ zu einem Patienten positioniert werden kann, bekannt.

In der WO 03/092518 A1 sind in Fig. 11.A-D verschiedene Klammervorrichtungen gezeigt, mit denen ein Trokar in einer entsprechenden Öffnung der geschlossenen Klammer fixiert werden kann. Das Instrument selbst kann dann entlang der Trokarachse, die als z-Achse bezeichnet wird, bewegt werden. In Fig. 11.E der WO 03/092518 A1 ist außerdem ein als Schnellverschluss ausgebildeter Kopplungsmechanismus zur Kopplung des Klammerelements an einen axial rotierbaren Schaft dargestellt. Der Kopplungsmechanismus umfasst eine Steckverbindung zwischen dem Klammerelement und dem rotierbaren Schaft sowie eine Gleithülse mit einer Halteposition, die Bereiche eines Schaftes des Klammerelements sowie des rotierbaren Schaftes umschließt. Der rotierbare Schaft ist mittels eines Kegelradgetriebes mit einem Motor verbunden und kann axial rotiert werden. Die in der WO 03/092518 A1 beschriebene Trokarhalterung ist allerdings nicht für den Einsatz in einem robotischen System gedacht, sondern dient nur der stabilen Positionierung eines Trokars bei laparoskopischen Operationen.

In der EP 3 111 876 A2 wird eine Klammervorrichtung beschrieben, welche mit einem robotischen Operationssystem verwendet werden kann. Die Klammervorrichtung hält im geschlossenen Zustand direkt einen Trokar bzw. Trokarhalter, zum Öffnen und Schließen der Klammer verfügt sie über einen Hebelmechanismus, wie beispielsweise in den Figuren 19 A und 19 B der EP 3 111 876 A2 demonstriert. Das Innere der Klammer kann mit einem sterilen Tuch ausgekleidet werden. Eine sichere Haltung ist nur für einen bestimmten Trokardurchmesser oder einen bestimmten Durchmesser einer Trokarhalterung möglich. Über ein Basiselement kann die Klammer mit einem Manipulatorarm verbunden werden. Der Trokar bzw. die Trokarhalterung kann in diesem Fall nur in axialer Richtung bewegt werden, alle anderen Einstellungen müssen mit Hilfe des Manipulatorarms selbst erfolgen, an dem das Basiselement fest befestigt ist. Eine Anpassung an verschiedene Trokardurchmesser ist in dem Sinne möglich, dass in der Klammer selbst nur der Kopf einer Trokarhalterung fixiert ist, an dem sich ein Tubus anschließt, welcher in der Regel einen wesentlich kleineren Durchmesser als der Kopf hat, so dass verschiedene Durchmesser realisiert werden können.

In der EP 3 103 410 A1 zeigen die Figuren 11, 12 und 15 einen Trokar 800, der an einer Trokarhalterung 17 kippbar gelagert ist. Eine Vorrichtung zum einfachen Wechseln des Trokars oder der Trokarhalterung ist nicht vorgesehen. Die Kippbarkeit ermöglicht jedoch eine kompaktere Bauweise und belastet das Gewebe eines Patienten weniger. Weiterer vorbekannter Stand der Technik wird in den folgenden Patentschriften offenbart: DE 10 2013 002813 A1, DE 10 2016 111737 A1, WO 00/18306 A1, WO 2013/075205 A1, EP 1 925 260 A2, US 2010/292724 A1.

### Beschreibung der Erfindung

Aufgabe der Erfindung ist es eine Trokarhalterung für ein robotisches Operationssystem der eingangs beschriebenen Art dahingehend weiterzuentwickeln, dass zum einen eine leichte Auswechselbarkeit gegeben ist, eine Anpassung an verschiedene Durchmesser von Trokaren auf einfachere Weise realisiert werden kann, wobei für alle Durchmesser ein gleichermaßen sicherer Halt gewährleistet werden soll, und dabei außerdem die Belastung des Patienten so gering wie möglich zu halten.

Erfindungsgemäß wird diese Aufgabe bei einer Trokarhalterung der eingangs beschriebenen Art dadurch gelöst, dass die Trokarhalterung ein Aufnahmeelement für einen Trokar umfasst.

Mit dem Klammermechanismus werden die Schenkel geöffnet und geschlossen, bei geöffneten Schenkeln lässt sich der Trokar samt Aufnahmeelement entfernen und beispielsweise austauschen. Auf diese Weise können Aufnahmeelemente für verschiedene Trokare ohne besonderen Aufwand gewechselt werden, beispielsweise kann ein Aufnahmeelement für einen Trokar, der ein Skalpell aufnehmen soll, gegen einen dickeren für ein Endoskop ausgetauscht werden. Da die Aufnahmeelemente an die diversen Trokare optimal angepasst werden können, ist immer der bestmögliche Halt gegeben. Die Aufnahmeelemente können insbesondere auch mit den Trokaren fest verbunden sein, und bevorzugt aus Kunststoff gefertigt sein.

Bevorzugt wird das Aufnahmeelement bei geschlossenen Schenkeln von diesen um eine in der Klammerebene liegende erste Rotationsachse rotierbar gehalten. Auf diese Weise wird ein Freiheitsgrad für die Trokarhalterung derart realisiert, dass diese um einen Pivotpunkt auf Ihrer Längsachse schwenkbar ist, wodurch ein kompakterer Aufbau ermöglicht sowie eine Reduzierung der Gewebebelastung des Patienten bei eingesetztem Trokar erreicht wird.

Entsprechend der Erfindung ist im Bereich der Spitzen jedes der beiden Schenkel ein sogenanntes Radiaxlager ausgebildet, d.h. ein Lager, welches für eine Welle oder Achse sowohl eine Radialbewegung verhindert, als auch eine axiale Bewegung in einer Richtung entlang der Achse, beispielsweise mit Hilfe eines Anschlags. Durch die Verwendung von zwei Radiaxlagern lässt sich die axiale Bewegung in beiden Richtungen entlang der Achse beschränken. Das Aufnahmeelement selbst ist in diesem Fall als Achse ausgebildet und wird von den beiden Radiaxlagern eingespannt, so dass das Aufnahmeelement bei geschlossenen Schenkeln von den Radiaxlagern an einer axialen Bewegung gehindert ist. Zur Durchführung des Trokars weist das Aufnahmeelement eine durchgehende Öffnung senkrecht durch die Achse auf. Bei der Öffnung kann es sich um eine einfache ringförmige Öffnung handeln, deren Innendurchmesser etwa dem Außendurchmesser des Trokars entspricht. Je nach Durchmessers des Trokars können verschiedene Aufnahmeelemente zum Einsatz können, die verschiedenen Durchmesser aufweisen. Auf diese Weise ist stets ein optimaler Halt des Trokars in dem Aufnahmeelement und damit in der Trokarhalterung gegeben, da mittels verschiedener Aufnahmeelemente eine Anpassung auf verschiedene Durchmesser exakt und ohne besonderen Aufwand realisiert werden kann. Dies erhöht die Sicherheit in der Handhabung des Operationssystems.

Übliche Trokare für robotische Operationssysteme verfügen über eine Instrumentenaufnahme mit einem eher großen Durchmesser und ein sich daran anschließendes Durchführungselement mit einem gegenüber der Instrumentenaufnahme kleineren Durchmesser; das Durchführungselement ist in der Regel als zylinderförmiger Tubus ausgestaltet. Das Instrument wird mindestens von der Instrumentenaufnahme daher an einem Durchrutschen gehindert, es wird darin gelagert und ggf. auch fixiert. Werden im Zusammenhang mit der Erfindung speziell angepasste Trokare verwendet, deren Tubus nicht zylinderförmig ausgebildet ist, sondern bei denen das Durchführungselement die Form eines Kegelstumpfes aufweist, der sich von der Instrumentenaufnahme abstehend - also von der Instrumentenaufnahme weg - verjüngt, wobei der Kegelwinkel äußerst schmal gewählt wird, so dass ein Unterschied zur Zylinderform mit bloßem Auge kaum erkennbar ist, so kann mittels des Durchmessers der durchgehenden Öffnung im Aufnahmeelement die Eindringtiefe des Trokars in begrenztem Umfang kontrolliert und zumindest begrenzt werden.

Der Kopplungsmechanismus für die Verbindung zwischen Basiselement und Klammerelement ist bevorzugt als Schnellverschluss ausgestaltet, der mit der Hand ohne weitere Hilfsmittel bedienbar ist. Beispielsweise eignet sich besonders gut eine Schnappverbindung. Das Basiselement wird dann fest am Manipulator montiert und das Klammerelement mittels der Schnappverbindung am Basiselement fixiert, eine Relativbewegung zwischen Basiselement und Klammerelement ist dann ausgeschlossen. Das Klammerelement selbst ist dann mechanisch, d.h. per Hand zu bedienen. Eine sterile Trennung zwischen dem Klammerelement und dem Basiselement kann erreicht werden, indem vor der Herstellung der Verbindung zwischen den beiden Elementen eine Sterilisationsfolie zwischen beide eingebracht wird. Diese kann beispielsweise zwischen den beiden Elementen geklemmt gehalten werden oder mit dem Klammerelement lösbar oder fest verbunden sein. Der Sterilitätsbereich kann dadurch letzten Endes gegenüber dem Stand der Technik weiter vom Patienten weggelegt werden, was die hygienische Sicherheit verbessert. Dafür müssen alle Teile auf der dem Patienten zugewandten Seite der Sterilisationsfolie steril ausgelegt sein, was mindestens das Aufnahmeelement, das Klammerelement und den Klammermechanismus umfasst; selbstverständlich auch den Trokar.

In einer besonders bevorzugten Ausgestaltung sind mindestens das Aufnahmeelement, das Klammerelement und das Basiselement aus Kunststoff gefertigt. Die Ausbildung aus Kunststoff ermöglicht es, die Kosten bei der Herstellung zu reduzieren und zumindest das Klammerelement und das Aufnahmeelement auch als Einwegteile, d.h. für den einmaligen Gebrauch, vorzusehen, was die hygienischen Bedingungen weiter verbessert.

Die beiden Schenkel und der Rücken des Klammerelements sind besonders bevorzugt einstückig ausgebildet. Insbesondere bei der einstückigen Fertigung von Schenkeln und Rücken, aber auch bei anderen Arten der Verbindung, beispielsweise einer Schraub- oder Klebeverbindung weist die aus Schenkeln und Rücken gebildete Klammer vorteilhaft eine Federwirkung auf, die dazu führt, dass die Schenkel bei elastischer Auslenkung aus einer Ruheposition, einer Nullstellung bei der keine Rückstellkraft wirkt, elastisch in diese zurückfedern, sofern die zur Auslenkung nötige Krafteinwirkung nachlässt bzw. sie nicht mehr in ihrer ausgelenkten Position gehalten werden. Mindestens einer der Schenkel ist daher vorteilhaft federnd mit dem Rücken verbunden. Die elastische Auslenkung aus der Nullstellung erfolgt mittels Krafteinwirkung, die beispielsweise durch den Klammermechanismus ausgeübt werden kann.

Der Klammermechanismus kann auf verschiedenen Weise realisiert werden, wobei darauf geachtet werden muss, dass das Aufnahmeelement mit dem Trokar sicher gehalten wird und vor unbeabsichtigtem Lösen geschützt ist. In einer zweckmäßigen Ausgestaltung, nicht Teil der hier beanspruchten Erfindung, umfasst der Klammermechanismus mindestens einen Stift, der in einem von Null verschiedenen Winkel zur Klammerebene im betreffenden Schenkel fixiert ist. Außerdem umfasst der Klammermechanismus dann ein entlang einer oder um eine Bewegungsachse relativ zum Klammerelement bewegliches Führungselement, in welches mindestens ein Langloch mit Schmalseiten und Längsseiten eingebracht ist. Die Anzahl der Langlöcher korrespondiert dabei zur Anzahl der Stifte, wobei in jedem der Schenkel ein Stift vorgesehen sein kann. Der mindestens eine Stift, der vorzugsweise aus Metall gefertigt ist, insbesondere Edelstahl, wird in dem mindestens einen Langloch geführt, wobei die beiden Schmalseiten des mindestens einen Langlochs in verschiedenen Abständen zur Bewegungsachse liegen, so dass sich der mindestens eine Schenkel - entsprechend der Anzahl der Stifte - bei Bewegung des Führungselements entlang der oder um die Bewegungsachse öffnet oder schließt. Bevorzugt werden zwei Stifte verwendet, d.h. in jedem der beiden Schenkel ist je ein Stift fixiert, vorzugsweise senkrecht zur Klammerebene. Dies erhöht die Stabilität und verbessert die Handhabung.

Durch die Bewegung des Führungselements erfolgt eine Relativbewegung zwischen Stift und Langloch, die durch die Schmalseiten des Langlochs beschränkt wird. Befindet sich der Stift an der näher zur Bewegungsachse liegenden Schmalseite, so ist die Klammer bzw. der betreffende Schenkel geschlossen. Befindet sich der Stift an der anderen Schmalseite des Langlochs, so ist die Klammer bzw. der Schenkel geöffnet.

Der Klammermechanismus kann dann auf verschiedene Arten realisiert werden. In einer möglichen Ausgestaltung liegt die Bewegungsachse in der Klammerebene und ist von beiden - in der Regel symmetrischen - Schenkeln gleich beabstandet, liegt also genau in der Mitte zwischen beiden. Das Führungselement ist in diesem Fall als Führungsschlitten ausgebildet, welcher entlang der Bewegungsachse verschiebbar ist. Das mindestens eine Langloch kann gerade ausgeführt werden, um eine in Bezug auf die Verschiebung, d.h. die zurückgelegte Strecke, lineare Öffnung bzw. Schließung der Klammer zu erreichen. Andere, gekrümmte Langlöcher sind möglich, um beispielsweise den Kraftaufwand bei zunehmender Öffnung zu verringern, was bedeutet, dass bei zunehmender Öffnung die Verschiebestrecke des Schlittens für einen vorgegebenen Winkel größer wird, da die Krümmung verringert wird.

Noch kompakter lässt sich der Klammermechanismus gestalten, wenn die Bewegungsachse als zweite Rotationsachse ausgebildet ist, welche mit der Klammerebene einen von Null verschiedenen Winkel, bevorzugt einen rechten Winkel einschließt. Das Führungselement ist in diesem Fall um die zweite Rotationsachse drehbar, wobei das mindestens eine Langloch -welches auch als Kulisse bezeichnet wird - mindestens teilweise gekrümmt ist. Die zweite Rotationsachse verläuft dabei bevorzugt durch das Führungselement.

Durch Drehung des Führungselements um die zweite Rotationsachse werden - bei der beispielhaften, aber nicht beschränkenden Verwendung zweier Stifte - die Stifte in den beiden Langlöchern zwischen den Endpositionen, d.h. den Schmalseiten der Langlöcher hin und her bewegt, je nach Rotationsrichtung. Diejenigen Schmalseiten der Langlöcher, die den kürzesten radialen Abstand zur zweiten Rotationsachse haben, definieren dann den Zustand einer geschlossenen Klammer bzw. geschlossener Schenkel, hier sind die Stifte der zweiten Rotationsachse des Führungselements am nächsten. Die anderen Schmalseiten, die von der zweiten Rotationsachse am weitesten entfernt liegen, definieren dann die Position der geöffneten Klammer bzw. geöffneter Schenkel. Dabei ist es nicht zwingend notwendig, dass beide Stifte jeweils die Endpositionen, d.h. die Schmalseiten auch tatsächlich erreichen, wesentlich ist, dass die Bewegung des Führungselements in einer Relativbewegung der beiden Schenkel aufeinander zu bzw. voneinander weg resultiert, wenn die Bewegungsrichtung umgekehrt wird.

Dabei reicht es prinzipiell aus, wenn nur ein Schenkel mit einem Stift versehen ist und das Führungselement ein einziges Langloch aufweist, in welchem der Stift geführt wird. Bereits auf diese Weise lässt sich die Klammer öffnen und schließen, indem der eine Schenkel in der Klammerebene von dem anderen weg bzw. auf diesen zu bewegt wird.

Vorteilhaft ist es jedoch, zwei Langlöcher mit zwei Stiften vorzusehen, dies ermöglicht zum einen die Klammer weiter zu öffnen, wenn die Abstände der Schmalseiten der Langlöcher in Bezug auf die Bewegungsachse gleich bleiben, im Vergleich der Verwendung nur eines Langlochs. Zum anderen wird dadurch die Stabilität des Systems erhöht und die Handhabung erleichtert, da zur Erzielung des gleichen Abstandes der Spitzen der Schenkel im Vergleich zur Bewegung nur eines Schenkels die Auslenkung der beiden Schenkel geringer ausfällt, so dass der Kraftaufwand verringert werden kann und die Materialermüdung durch die elastische Verformung beim Aufbiegen reduziert wird.

Zweckmäßig und besonders einfach zu bedienen ist dabei ein Klammermechanismus, bei dem die Stifte - und für den Fall, dass die Bewegungsachse auch als Rotationsachse ausgebildet ist, auch die Rotationsachse - senkrecht zur Klammerebene angeordnet sind.

Um zu vermeiden, dass sich der Klammermechanismus unwillkürlich öffnet oder schließt, wenn der Trokar beispielsweise bewegt wird, kann der mindestens eine Stift in Bezug auf das mindestens eine Langloch eine Übermaßpassung aufweisen, die so bemessen ist, dass eine mechanische Verschiebung unter geringem Krafteinsatz ohne Anstrengung eines Bedieners dennoch möglich bleibt.

In dem Falle, dass die Bewegungsachse eine zweite Rotationsachse ist, um die das Führungselement rotierbar ist, lässt sich letzteres besonders vorteilhaft auf einer mit dem Klammerelement verbundenen Zunge, die besonders zweckmäßig am Rücken des Klammerelements befestigt ist, rotierbar befestigen. Die Zunge kann einstückig mit dem Rücken ausgebildet sein. Das rotierbare Führungselement hat gegenüber dem verschiebbaren den Vorteil, dass es immer im gleichen Bereich des Klammerelements angeordnet ist, wodurch eine kompakte Anordnung und eben auch die Befestigung am Klammerelement selbst auf einfache, kompakte Weise möglich ist, diese Befestigung fixiert die zweite Rotationsachse zusätzlich und stabilisiert die Vorrichtung weiter.

In einer weiteren bevorzugten Ausgestaltung weist das mindestens eine Langloch im Führungselement im Bereich der weiter von der Bewegungsachse entfernt liegenden Schmalseite an derjenigen Längsseite, die der Bewegungsachse zugewandt ist, eine Ausbuchtung in Richtung der Bewegungsachse auf, in welche der mindestens eine Stift bei geöffneten Schenkeln einrastet.

Dies ist dann besonders sinnvoll, wenn Rücken und Schenkel einstückig gefertigt sind und aufgrund der elastischen Biegung beim Öffnen der Schenkel die Stifte aufgrund der Rückstellkraft in die Ausbuchtungen gedrückt werden. Die geöffnete Stellung wird damit besser fixiert und die Handhabung beim Wechsel von Aufnahmeelementen sicherer.

Zweckmäßig weist das Führungselement dabei, insbesondere wenn die Bewegungsachse als zweite Rotationsachse ausgebildet ist, einen Hebel zum Betätigen des Klammermechanismus auf. Der Hebel greift dann an der einen Seite an die Rotationsachse an und erstreckt sich in im Wesentlichen radialer Richtung von ihr weg. Beim Betätigen des Hebels wird also das Führungselement um die Rotationsachse gedreht.

Bevorzugt sind dabei am Hebel und am Klammerelement einander entsprechende, d.h. zueinander korrespondierende Fixierungsmittel ausgebildet, beispielsweise als Rastverbindung, mittels welcher der Hebel mindestens bei geschlossenen Schenkel in seiner Position gesichert ist. Dies erschwert zusätzlich die Möglichkeit des unwillkürlichen Öffnens, sichert die Trokarhalterung aber auch gegen unbeabsichtigtes Öffnen bei zufälliger Berührung.

Vorzugsweise befindet sich der Hebel dann in einer projizierten Draufsicht auf die Klammerebene innerhalb der Kontur des Klammerelements oder schließt mit diesem bündig ab, was ebenfalls eine zusätzliche Sicherung gegen unbeabsichtigtes Öffnen darstellt.

In einer weiteren Ausgestaltung ist das Aufnahmeelement fest mit einem Trokar (18) verbunden und bevorzugt einstückig mit diesem gefertigt. Damit kann ein versehentliches Herausrutschen des Trokars aus dem Aufnahmeelement verhindert werden.

Eine Abwandlung des Klammermechanismus mit einem um die zweite, mit der Klammerebene einen von Null verschiedenen Winkel einschließende Rotationsachse drehbaren Führungselement ergibt sich, wenn anstelle von Stiften an dem Führungselement mindestens ein bezüglich der zweiten Rotationsachse exzentrisch angeordnetes Exzenterelement angebracht ist, so dass sich bei einer Drehung des Führungselements um die zweite Rotationsachse mindestens einer der Schenkel durch die Wirkung des Exzenterelements öffnet bzw. schließt. Das Exzenterelement kann dabei gesondert auf dem - bevorzugt scheibenförmig ausgebildeten - Führungselement angeordnet oder ausgebildet sein, es kann aber auch Teil des Hebelgriffs sein, indem dieser in dem Bereich, in welchem er mit dem Führungselement verbunden ist, eine entsprechend variierende Krümmung aufweist. Im geschlossenen Zustand steht das mindestens eine Exzenterelement dann nicht in Kontakt mit dem entsprechenden Schenkel oder liegt an diesem nur an, so dass der Schenkel nicht unter Vorspannung steht. Durch Drehung des Führungselements wird das Exzenterelement dann in den Bereich des Schenkels geführt und drückt diesen in der Klammerebene von der zweiten Rotationsachse weg. Vorzugsweise werden auch hier zwei Exzenterelemente verwendet, die jedoch einstückig mit dem Hebel ausgebildet sein können.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

### Kurze Beschreibung der Zeichnungen

Nachfolgend wird die Erfindung beispielsweise anhand der beigefügten Zeichnungen, die auch erfindungswesentliche Merkmale offenbaren, noch näher erläutert. Es zeigen:
- Fig. 1a), b): die Gesamtansicht eines robotischen Operationssystems,
- Fig. 2: eine Instrumententrägervorrichtung mit einer Trokarhalterung und Trokar in Perspektivansicht,
- Fig. 3: eine Trokarhalterung mit Trokar in Perspektivansicht,
- Fig. 4: eine Explosionszeichnung der Trokarhalterung mit Trokar,
- Fig. 5a): ein Klammerelement der Trokarhalterung von unten,
- Fig. 5b): ein Aufnahmeelement der Trokarhalterung von unten,
- Fig. 5c): ein Führungselement der Trokarhalterung von unten,
- Fig. 6a), b): die Trokarhalterung im geschlossenen Zustand von oben bzw. unten,
- Fig. 7: das Klammerelement mit einem eingesetzten Aufnahmeelement im geschlossenen Zustand,
- Fig. 8: das Klammerelement mit daraus entferntem Aufnahmeelement im geöffneten Zustand, und
- Fig. 9: eine Trokarhalterung mit Trokar und Sterilisationsfolie.

### Ausführliche Beschreibung der Zeichnungen

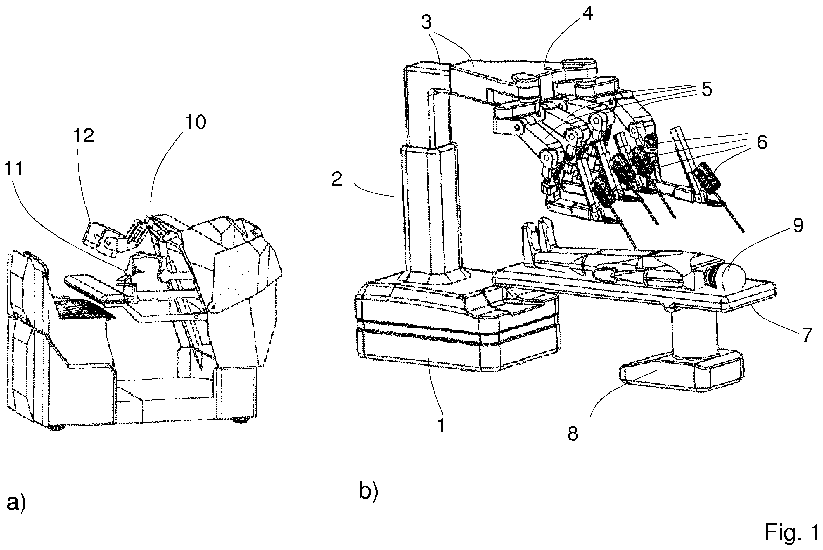

Fig. 1 zeigt zunächst ein robotisches Operationssystem in einer Übersichtsdarstellung. In Fig. 1b) ist die Trägerstruktur des robotischen Operationssystem gezeigt, die auf einem Standfuß 1 ruht, auf dem eine in ihrer Höhe einstellbare Tragsäule 2 mit einem davon abstehenden Ausleger 3 sowie einer Gabelstruktur 4 am freien Ende des Auslegers 3 angeordnet sind. Am Ausleger 3 mit der Gabelstruktur 4 sind beispielhaft insgesamt vier Manipulatoren 5 mit Instrumentenhaltern 6 angeordnet. Unterhalb der Manipulatoren 5 mit Instrumentenhaltern 6 ist eine Patientenliege 7 auf einem Liegenstandfuß 8 angeordnet, auf der sich ein zu operierender Patient 9 befindet. Die Patientenliege 7 kann auf dem Liegenstandfuß 8 drehbar gelagert sein. Die Trägerstruktur des robotischen Operationssystems und die Patientenliege 7 können vorteilhaft relativ zueinander auch translatorisch bewegt werden.

Das robotische Operationssystem verfügt auch über eine Steuervorrichtung 10, die in Fig. 1a) gezeigt ist und gegenüber der Trägerstruktur ebenfalls relativ bewegbar, hier verfahrbar ist. Sie wird von einem speziell ausgebildeten Chirurgen bedient, und die Bewegungen des Chirurgen an Handgriffen 11 der Steuervorrichtung 10 werden auf die Manipulatoren 5 bzw. auf in den Instrumentenhalterungen 6 gehaltene Instrumente übertragen. Der Chirurg kann dabei mit Hilfe einer Betrachtungseinrichtung 12 das Operationsfeld überwachen.

Bei dem Manipulator 5 handelt es sich um ein vielgliedriges System, welches einer losen kinematischen Kette entspricht. Ein Teil der Glieder bildet eine Positionierungsvorrichtung - auch als Manipulatorarm bezeichnet - und der übrige Teil der Glieder eine Instrumententrägervorrichtung, der Instrumentenhalter 6 ist an letzterer montiert.

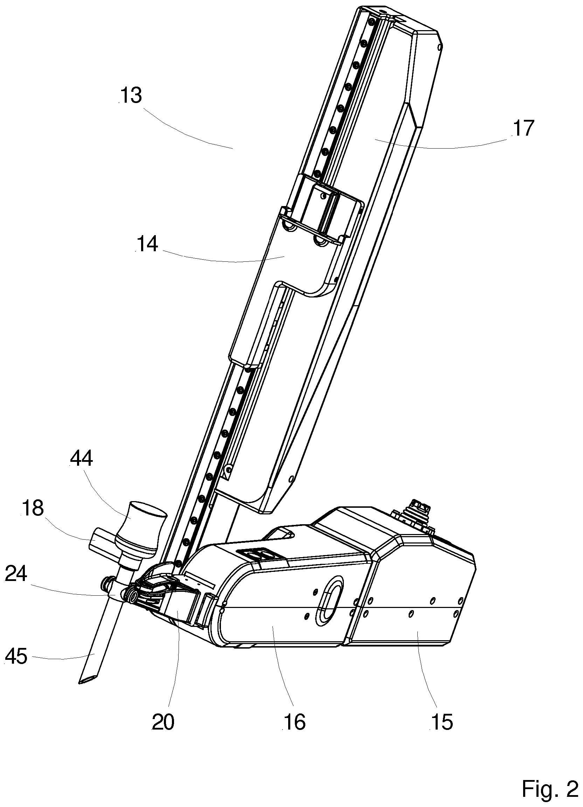

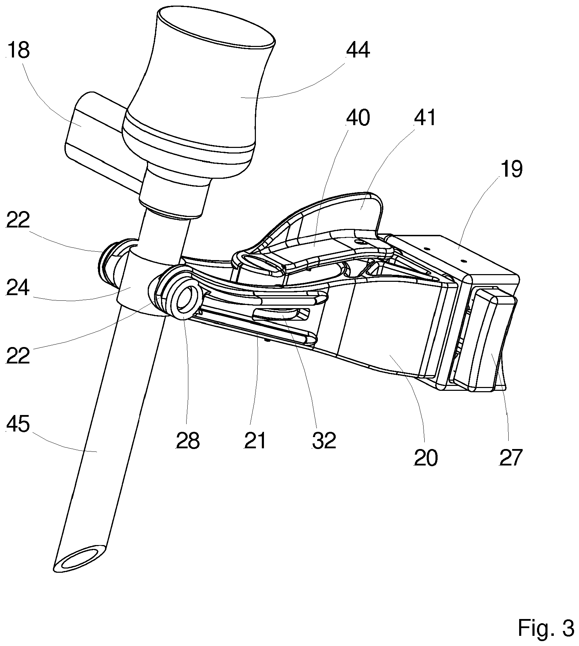

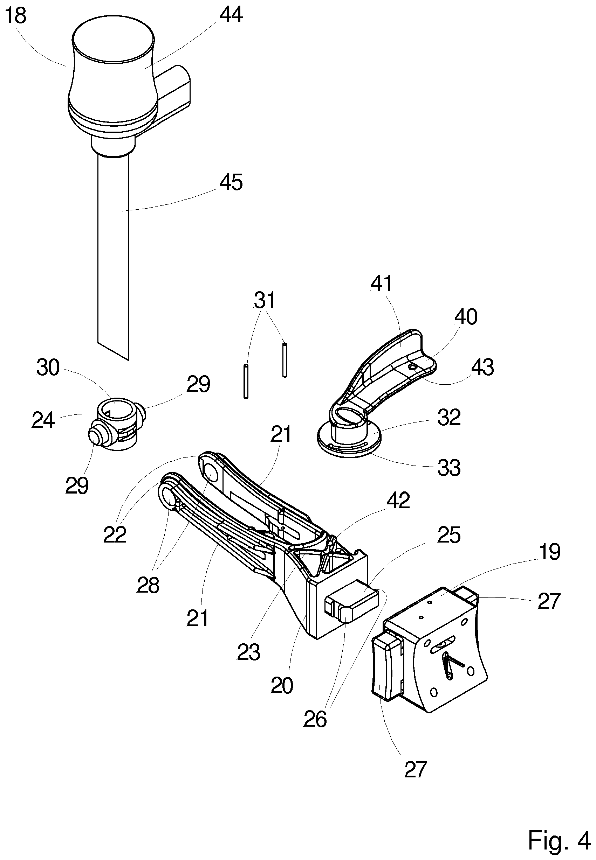

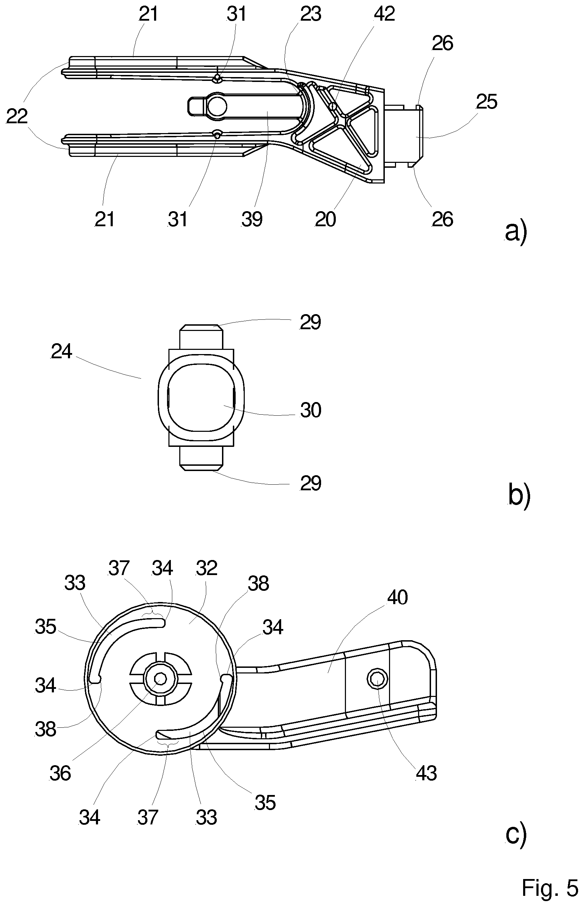

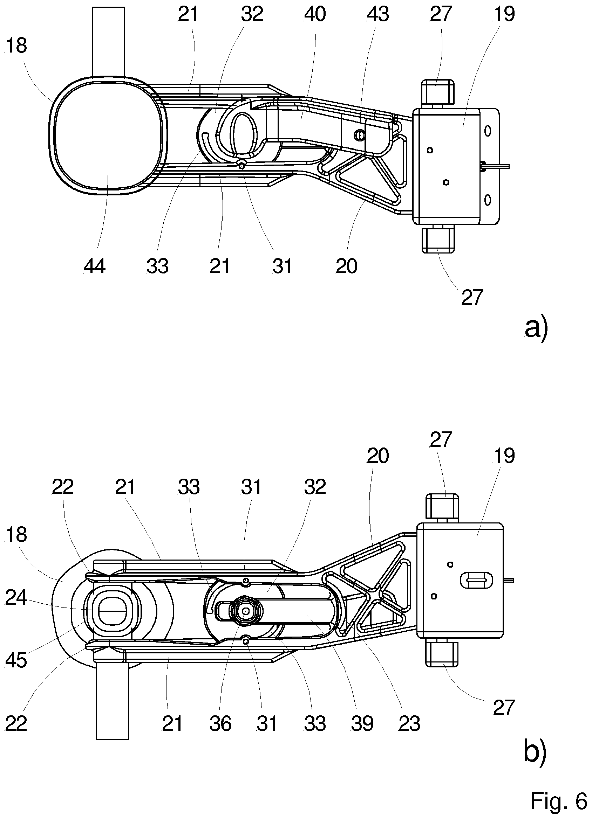

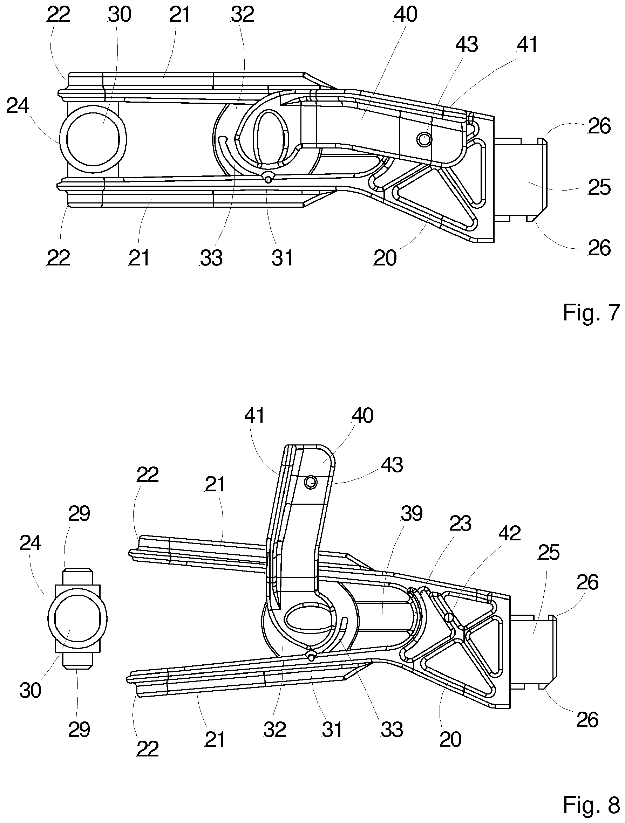

Fig. 2 zeigt eine solche Instrumententrägervorrichtung 13, allerdings ohne Instrumentenhalter 6. Der Instrumentenhalter 6 wird an die Instrumententrägervorrichtung 13 über einen translatorisch verfahrbaren Schlitten 14 angekoppelt. Die Instrumententrägervorrichtung 13 umfasst ein erstes Armglied 15, mit welchem die Instrumententrägervorrichtung 13 an die Positioniervorrichtung des Manipulators 5 angekoppelt wird. Sie umfasst weiterhin ein zweites Armglied 16, welches gegenüber dem ersten Armglied 15 um eine gemeinsame Längsachse rotierbar ist. Das zweite Armglied 16 verbindet das erste Armglied 15 mit einem dritten Armglied 17, welches um eine zu der gemeinsamen Längsachse des ersten und zweiten Armgliedes senkrecht orientierte Achse drehbar ist. In der Verlängerung von erstem Armglied 15 und zweitem Armglied 16 ist an der Spitze des zweiten Armgliedes 16 eine Trokarhalterung mit einem Trokar 18 angebracht, die Trokarhalterung wird im Folgenden im Wesentlichen anhand der Perspektivansichten der Figuren 3 und 4 näher erläutert. Die Fig. 5a)-c) zeigen einzelne Teile der Trokarhalterung im Detail, Fig. 6a)-b) die geschlossene Trokarhalterung in zwei Ansichten, während Fig. 7 und Fig. 8 das Öffnen und Schließen der Trokarhalterung demonstrieren.

Fig. 3 zeigt die Trokarhalterung aus Fig. 2 mit einem Trokar 18, jedoch ohne Instrumententrägervorrichtung 13. Die Trokarhalterung umfasst ein Basiselement 19 zur Befestigung der Trokarhalterung am Manipulator 5 bzw. der Instrumententrägervorrichtung 13 des Manipulators 5. Die Trokarhalterung umfasst außerdem ein Klammerelement 20, welches über einen Kopplungsmechanismus mit dem Basiselement 19 auswechselbar verbunden ist. Am Klammerelement 20 sind zwei relativ zueinander in einer Klammerebene bewegliche Schenkel 21 mit freien Spitzen 22 ausgebildet. Außerdem verfügt das Klammerelement 20 über einen Klammermechanismus zum Öffnen und Schließen der Schenkel 21. Die Schenkel 21 sind an ihren den Spitzen 22 gegenüber liegenden Enden mittels eines Rückens 23 verbunden. Schließlich umfasst die Trokarhalterung ein Aufnahmeelement 24 für den Trokar 18, das Aufnahmeelement 24 wird bei geschlossenen Schenkeln 21 von diesen um eine in der Klammerebene liegende erste Rotationsachse rotierbar gehalten wird.

Der Kopplungsmechanismus zur Verbindung des Basiselements 19 mit dem Klammerelement 20 ist mit der Hand ohne weitere Hilfsmittel bedienbar und als Schnellverschluss, hier als Schnappverbindung ausgebildet. Am Klammerelement 20 befindet sich dazu ein Schnappelement 25, an welchem eine oder mehrere Schnappnasen 26 ausgebildet sind. Am Basiselement 19 befindet sich eine - hier nicht dargestellte - Öffnung, in welche das Schnappelement 25 eingesetzt wird und entsprechend einrastet. Zum schnellen Lösen der Rastverbindung befinden sich am Basiselement 19 zwei mechanischen Drucktasten 27, die zum Lösen der Schnappverbindung zusammengedrückt werden. Dies kann mit einer Hand erfolgen, die andere Hand kann das Klammerelement 20 halten. Vor dem Betrieb wird, bevor Klammerelement 20 und Basiselement 19 zusammengesetzt werden, zwischen beiden eine Sterilisationsfolie gelegt, die von der Schnappverbindung im zusammengesetzten Zustand fixiert wird.

Die beiden Schenkel 21 sind relativ zueinander in einer Klammerebene beweglich. Die Klammerebene ist jene Ebene, in der die beiden Schenkel liegen und in der auch mittels des Klammermechanismus die Relativbewegung der Schenkel erzeugt wird. Mindestens einer der beiden Schenkel wird also bei Betätigung des Klammermechanismus bewegt.

Auch die erste Rotationsachse, um die der Trokar 18 rotieren kann, liegt in der Klammerebene. Um die Rotation zu ermöglichen, ist erfindungsgemäss im Bereich der Spitzen 22 jedes der beiden Schenkel 21 ein Radiaxlager 28 ausgebildet. Das Aufnahmeelement 24 ist als Achse ausgebildet und wird bei geschlossenen Schenkeln von den Radiaxlagern 28 an einer axialen Bewegung gehindert. Dazu liegt es mit Achsendbereichen 29 in den Radiaxlagern 28. Außerdem weist das Aufnahmeelement 24 zur Durchführung des Trokars 18 senkrecht durch die Achse eine durchgehende Öffnung 30 auf. In diese wird der Trokar 18 eingeschoben. Das Aufnahmeelement 24 ist auswechselbar und kann in verschiedenen Größen, d.h. mit Öffnungen verschiedener Durchmesser bereitgestellt werden. Auf diese Weise ist immer eine optimale Anpassung an den jeweiligen Trokar 18 möglich, da für verschiedene Instrumente verschiedene Trokare verwendet werden können. Trokare für Endoskope sind beispielsweise etwas dicker als Trokare für Scheren oder Skalpelle.

Der Trokar 18 weist eine Instrumentenaufnahme 44 auf, an die sich davon abstehend ein Durchführungselement 45 anschließt. Das Durchführungselement 45 weist dabei eine sich von der Instrumentenaufnahme 44 abstehend verjüngende Form eines Kegelstumpfes auf. Der Kegelwinkel ist extrem spitz, bevorzugt weniger als 1°, besonders bevorzugt weniger als 0,1°; auf diese Weise kann eine entsprechende Eindringtiefe vorgegeben werden: je spitzer der Kegelwinkel, desto höher die Eindringtiefe, wenn das gleiche Aufnahmeelement 24 verwendet wird. Da die Kegelform sehr spitz ist, wird dennoch ein fester Sitz des Trokars 18 im Aufnahmeelement 24 ermöglicht.

Die Schenkel und der Rücken des Klammerelements sind bevorzugt einstückig ausgebildet und sind bevorzugt aus Kunststoff gefertigt. Dies ermöglicht zum einen geringe Herstellungskosten und zum anderen die Auslegung des Klammerelements als Einwegartikel, der nach jeder Operation entsorgt wird, was hygienisch vorteilhaft ist.

Insbesondere bei der einstückigen Fertigung von Schenkeln 21 und Rücken 23, aber auch bei anderen Arten der Verbindung, beispielsweise einer Schraub- oder Klebeverbindung weist die aus Schenkeln und Rücken gebildete Klammer bevorzugt eine Federwirkung auf, die dazu führt, dass die Schenkel 21 bei elastischer Auslenkung aus einer Ruheposition, die hier beispielsweise die in Fig. 5a) dargestellte sein kann, elastisch in diese zurückfedern, sofern die zur Auslenkung nötige Krafteinwirkung nachlässt bzw. sie nicht mehr in ihrer ausgelenkten Position gehalten werden. Mindestens einer der Schenkel 21 ist daher zur Erzeugung dieser Federwirkung vorteilhaft federnd mit dem Rücken 23 verbunden. Die Ruheposition kann dabei grundsätzlich frei gewählt werden und ist beispielsweise beim Spritzguss keinen Beschränkungen unterworfen, d.h. die Schenkel müssen in der Ruheposition nicht zwingend, wie in Fig. 5a) gezeigt, parallel liegen, sondern können zu ihren Spitzen 22 hin auch zusammen oder leicht auseinander laufen.

Bei einer Federwirkung der Klammer kann das Aufnahmeelement 24 grundsätzlich auch von Hand in die Radiaxlager 28 an den Spitzen 22 der Schenkel 21 eingesetzt werden, dies ist jedoch aufwendig und umständlich, bei unsachgemäßer Handhabung kann das Klammerelement 21 dabei auch zerstört werden.

Um das Einsetzen des Aufnahmeelements 24 in das Klammerelement 20 zu erleichtern, weist die Trokarhalterung daher auch einen Klammermechanismus zum Öffnen und Schließen der Schenkel auf. Ein nicht-erfindungsgemässer Klammermechanismus umfasst mindestens einen Stift 31, der in einem von Null verschiedenen Winkel zur Klammerebene in einem Schenkel 21 fixiert ist. In den Zeichnungen sind beispielhaft zwei Stifte 31 gezeigt, die in von Null verschiedenen Winkeln zur Klammerebene in den Schenkeln 21 fixiert sind, in jedem Schenkel 21 ist ein Stift 31, der beispielsweise aus Edelstahl gefertigt sein kann, fixiert. Diese Stifte 31 sind in der Explosionszeichnung in Fig. 4 außerhalb der Schenkel 21 gezeigt, in Fig. 5a), Fig. 6a-b), Fig. 7 und Fig. 8 sind die Stifte 31 in die Schenkel 21 eingesetzt und dort fixiert. Die Fixierung kann kraftschlüssig, formschlüssig und /oder stoffschlüssig erfolgen. Im hier gezeigten Fall sind die Stifte 31 kraft- und formschlüssig mit den Schenkeln 21 verbunden, sie weisen gegenüber den Öffnungen in den Schenkeln 21, in die sie eingetrieben werden, eine leichte Übermaßpassung auf.

Der Klammermechanismus umfasst außerdem ein Führungselement 32, in welches eine Anzahl der Stifte 31 entsprechende Anzahl von Langlöchern 33 mit Schmalseiten 34 und Längsseiten 35 eingebracht sind. Im gezeigten Beispiel weist das Führungselement 32 daher zwei Langlöcher 33 auf, das Führungselement 32 ist in Fig. 5c) in einer Ansicht von unten dargestellt. Das Führungselement 32 ist in Bezug auf eine Bewegungsachse beweglich, die beiden Stifte 31 werden in den Langlöchern 33 geführt. Ein Öffnen und Schließen der Schenkel 21 wird erreicht, indem die beiden Schmalseiten 34 eines jeden Langlochs 33 in verschiedenen Abständen zu der Bewegungsachse liegen. Bei der Bewegungsachse kann es sich um eine Verschiebeachse handeln, die in der Klammerebene liegt und von beiden Schenkeln 21 gleich beabstandet ist, d.h. einen Punkt in der Mitte des Rückens sowie einen Punkt auf der Hälfte des Abstandes zwischen den beiden Spitzen 22 schneidet, also parallel zu den Schenkeln 21 liegt. Das Führungselement ist dann beispielsweise als Schlitten ausgestaltet und eine Bewegung des mindestens einen Stiftes 21 relativ zum Langloch 33 von der näher an der Bewegungsachse liegenden Schmalseite zur entfernter von der Bewegungsachse liegenden Schmalseite öffnet die Schenkel 21 bzw. die Klammer, eine entgegengesetzte Bewegung schließt die Klammer.

Eine andere Möglichkeit, die eine noch kompaktere und stabilere Bauform ermöglicht und die in den in den Zeichnungen dargestellten Beispielen realisiert ist, besteht darin, die Bewegungsachse als zweite Rotationsachse 36 auszugestalten, diese zweite Rotationsachse liegt senkrecht zur Klammerebene, in den Fig. 5-8 senkrecht zur Blattebene. Das mindestens eine Langloch 33 ist dann mindestens teilweise gekrümmt. Auch hier liegen die beiden Schmalseiten 34 eines jeden Langlochs 35 in verschiedenen Abständen zur zweiten Rotationsachse, eine Drehung des Führungselements 32 - gezeigt beispielsweise in Fig. 5c) - bewirkt eine Relativbewegung zwischen den Stiften 31 und den Langlöchern 33 zwischen den Schmalseiten 34, und durch die verschiedenen Abstände der Schmalseiten 34 in Bezug auf die zweite Rotationsachse 36 wird bei Drehung des Führungselements 32 um die Rotationsachse 36 eine Öffnung der Klammer bzw. eine Schließung der Klammer durch die entsprechende Bewegung der Schenkel 21 erreicht; die zweite Rotationsachse verläuft durch das Führungselement 32.

Im nicht ausgelenkten Zustand, d.h. im geschlossenen Zustand sind die Schenkel 21 in ihrer Ruheposition, d.h. es wirken keine strukturbedingten, internen Rückstellkräfte auf sie. Um das Schließen der Klammer zu erleichtern ist es daher vorteilhaft, wenn der Bereich des mindestens einen Langlochs 33, der näher an derjenigen Schmalseite 34 liegt, welche wiederum näher an der zweiten Rotationsachse 36 liegt und den geschlossenen Zustand der Klammer definiert, als gerader Abschnitt 37 des Langlochs 33 ausgeführt ist, dieser gerade Abschnitt 37 weist keine Krümmung auf und wirkt als sogenannter toter Bereich, da in diesem Bereich keine Verstellung stattfindet. Auf diese Weise lassen sich mögliche Fertigungstoleranzen berücksichtigen und es kann sichergestellt werden, dass das Führungselement 32 in eine Ruheposition gebracht und dort fixiert werden kann. Die Stifte 31 sind hier senkrecht zur Klammerebene angeordnet, ebenso wie die zweite Rotationsachse 36. Die Stifte 31 können gegenüber den Langlöchern 33 eine geringe Übermaßpassung aufweisen, um ein unwillkürliches Verstellen zu verhindern. Alternativ oder ergänzend kann das Führungselement 32 in den beiden Endpositionen, die die maximal mögliche Öffnung und die geschlossene Klammer definieren, auch fixiert werden. Um die Klammer im geöffneten Zustand zu fixieren und ein sicheres Auswechseln des Aufnahmeelements 24 zu ermöglichen, weisen die näher an der Bewegungsachse liegenden Langlöcher 33 im Bereich derjenigen Schmalseiten 34, die zur geöffneten Klammer korrespondieren, eine Ausbuchtung 38 auf, in welche der jeweilige Stift 31 auf Grund der intrinsischen Rückstellkraft der Klammer einrasten kann. Auch im Bereich der anderen Schmalseite 34 ist es möglich, eine solche Ausbuchtung vorzusehen, wenn die Schenkel 21 des Klammerelements 20 immer vorgespannt bleiben sollen, also vom Führungselement 32 daran gehindert werden, in eine Ruhestellung, bei der keine Rückstellkraft wirkt, zu gelangen. In diesem Fall kann auch der gerade Abschnitt 37 des Langlochs 33 entfallen.

In der hier gezeigten Ausführung ist das Führungselement 32 auf einer mit den Klammerelement 20 verbundenen Zunge 39 - dargestellt beispielsweise in Fig. 5a), Fig. 6b) und Fig. 8 - rotierbar befestigt. Durch die Verwendung der Zunge 39 wird die Stabilität der Trokarhalterung wesentlich erhöht und das Drehen des Führungselements 32 erleichtert, da die Fixierung auf der Zunge 39 auch einer Verkantung vorbeugt. Die Zunge 39 ist bevorzugt am Rücken 23 des Klammerelements 20 und bevorzugt mit diesem einstückig ausgebildet, so dass das Führungselement 32 ortsfest in Bezug auf den Rücken 23 bleibt, wenn die Schenkel 21 geöffnet oder geschlossen werden.

Insbesondere bei einem Führungselement 32, wie es hier u.a. in Fig. 5c) gezeigt ist und welches um eine zweite Rotationsachse 36 gedreht wird, ist es vorteilhaft, wenn das Führungselement 32 einen Hebel 40 zum Betätigen des Klammermechanismus aufweist, da dann der vom Bediener aufzuwendende Kraftaufwand verringert wird und die Bedienung generell vereinfacht wird. Grundsätzlich ist es auch möglich, beispielsweise den Durchmesser des hier scheibenförmig ausgeführten Führungselements 32 so zu erhöhen, dass es sich noch deutlicher als in Fig. 3 gezeigt über den Abstand der beiden Schenkel 21 hinaus erstreckt und ein Benutzer es von außen drehen kann, in dem er an den Umfang des Führungselements 32 angreift, der dazu mit Rillen oder anderen Elementen, die helfen, das Führungselement 32 besser zu greifen, versehen sein kann.

In der hier gezeigten Ausführung weist das Führungselement 32 einen Hebel 40 auf. Fig. 7 und Fig. 8 zeigen Fig. 7 das Klammerelement 20 von oben mit geschlossener Stellung des Hebels und Fig. 8 das Klammerelement 20 mit geöffneter Stellung des Hebels 40 und der Schenkel 21. Fig. 5c) zeigt das Führungselement 32 mit daran angebrachtem Hebel 40 in einer Ansicht von unten. Auch in der Explosionszeichnung Fig. 4 ist der Hebel 40 zu erkennen. Am Hebel 40 ist ein Griffelement 41 ausgebildet, welches insbesondere in den perspektivischen Zeichnungen Fig. 3 und Fig. 4 zu erkennen ist. Damit wird die Bedienung des Hebels 40 erleichtert.

Um das Führungselement 32 in der geöffneten Position zu fixieren, weisen die Langlöcher an ihrer Innenseite, d.h. an derjenigen Längsseite 35, die der Bewegungsachse - hier der zweiten Rotationsachse 36 - näher liegen, Ausbuchtungen 38 im Bereich der entsprechenden Schmalseiten 34, die dem geöffneten Zustand entsprechen, auf, wie oben bereits beschrieben. Entsprechende Ausbuchtungen 38 lassen sich alternativ oder ergänzend auch für den geschlossenen Zustand an der andern Schmalseite 34 und der anderen Längsseite 35 anbringen, wobei ein Halt darin nur dann gesichert ist, wenn die Klammer unter Vorspannung steht.

Ergänzend oder alternativ sind vorteilhaft am Hebel 40 und am Klammerelement 20 Fixierungsmittel ausgebildet oder angeordnet, die bevorzugt als Rastverbindung ausgebildet sind, mittels welcher der Hebel 40 mindestens bei geschlossenen Schenkeln 21 in seiner Position gesichert ist. Beispielhaft umfasst die Rastverbindung hier eine auf dem Klammerelement im Bereich des Rückens 23 angeordnete Rastnase 42 und ein am Hebel 40 ausgebildetes, zur Rastnase 42 korrespondierendes Rastloch 43. Auf diese Weise wird der Hebel 40 und damit auch das Führungselement 32 gegen unwillkürliches Öffnen bei geschlossener Klammer gesichert, was insbesondere dann vorteilhaft ist, wenn die Klammer nicht unter Vorspannung steht.

Alternativ zu der in Fig. 7 und Fig. 8 gezeigten Ausführung des Klammermechanismus ist es auch möglich, statt der Stifte 31 Exzenterelemente zu verwenden, welche bezüglich der zweiten Rotationsachse 35 auf dem Führungselement 22 exzentrisch angeordnet sind. Diese können gesondert auf dem Führungselement 22 ausgebildet sein und beispielsweise die Form von Zylindern aufweisen, die beispielsweise im Bereich derjenigen Schmalseiten 34 angeordnet sind, welche weiter weg von der zweiten Rotationsachse 36 liegen. Sie können aber auch der Form der Langlöcher 33 folgen und als gekrümmte, längliche Erhebungen ausgebildet sein. Schließlich können sie auch in den Bereich des Hebels 40 integriert sein, welcher den Hebel 40 mit dem Führungselement 22 verbindet, d.h. der Bereich um die zweite Rotationsachse 36. Die hier in Fig. 7 und Fig. 8 senkrecht zur Blattebene stehenden Rundungen bzw. gerundete Flächen dieses Bereichs können dann mit entsprechenden Krümmungen und größer ausgebildet werden. Bei allen Ausführungen der Exzenterelemente werden bei einer Drehung des Führungselements 22 um die zweite Rotationsachse 36 die Schenkel 31 durch die Wirkung des Exzenterelements geöffnet bzw. geschlossen.

Diese letztgenannte Ausführung lässt sich auch mit der Ausführung mit Stiften kombinieren, um eine höhere Sicherheit zu gewährleisten. Ansonsten kann bei der Ausführung mit Exzenterelementen auch auf die Stifte 31 verzichtet werden, was die Herstellung vereinfacht und kostengünstiger macht, da für den Klammermechanismus dann keine Metallteile mehr benötigt werden.

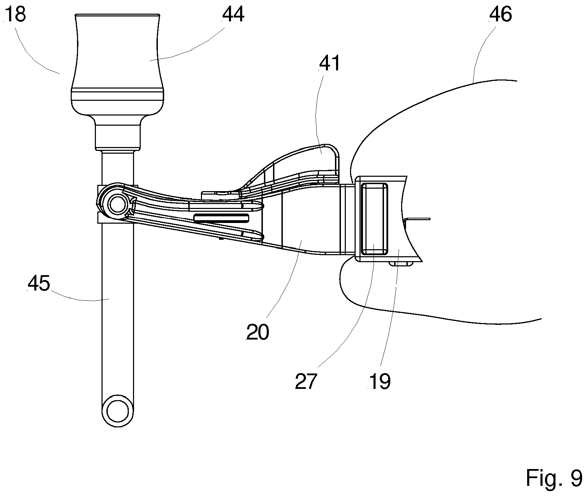

Fig. 9 schließlich zeigt die Trokarhalterung mit einer Sterilisationsfolie 46, welche zwischen dem Basiselement 19 und dem Klammerelement 20 angeordnet ist. Diese kann vor dem Betrieb wird, bevor Klammerelement 20 und Basiselement 19 zusammengesetzt werden, zwischen beide gelegt werden, die Sterilisationsfolie 46 wird dann von der Schnappverbindung im zusammengesetzten Zustand fixiert. Die Sterilisationsfolie 46 kann aber auch unlösbar mit dem Klammerelement 20 verbunden sein, wenn dieses - in Kombination mit dem Aufnahmeelement 24 und dem Trokar 18 sowie dem Klammermechanismus - als Einwegelement ausgelegt ist und bei der Herstellung steril verpackt wurde.

Die vorangehend beschriebene Trokarhalterung ermöglicht aufgrund ihrer einfach zu bedienenden Klammerfunktion ein einfaches Wechseln des Aufnahmeelements 24 und somit einen einfachen Wechsel zwischen Trokaren 18 verschiedener Durchmesser. Durch den zusätzlichen Kippfreiheitsgrad für den Trokar 18 wird die Belastung der Gewebedecke des Patienten reduziert, so dass das die Trokarhalterung führende Gerät kompakter ausgelegt werden kann. Eine Sterilitätsgrenze wird zwischen den Klammerelement 20 und dem Basiselement 19 durch Verwendung einer Sterilisationsfolie hergestellt, so dass hier der Sterilitätsbereich eine sehr große Ausdehnung in Bezug auf die Entfernung vom Patienten hat, was größtmögliche Sicherheit garantiert. Insbesondere wird das Klammerelement 20, aber auch das Basiselement 19 sowie weitere wichtige Teile wie Aufnahmeelement 24, Führungselement 32, Hebel 40 etc. aus Kunststoff gefertigt, so dass eine kostengünstige Herstellung möglich ist und das Klammerelement 20 auch als Einwegelement ausgelegt werden kann, was die Sicherheit zusätzlich erhöht.

### Bezugszeichenliste

- 1: Standfuß
- 2: Tragsäule
- 3: Ausleger
- 4: Gabelstruktur
- 5: Manipulator
- 6: Instrumentenhalter
- 7: Patientenliege
- 8: Liegenstandfuß
- 9: Patient
- 10: Steuervorrichtung
- 11: Handgriff
- 12: Betrachtungseinrichtung
- 13: Instrumententrägervorrichtung
- 14: Schlitten
- 15: erstes Armglied
- 16: zweites Armglied
- 17: drittes Armglied
- 18: Trokar
- 19: Basiselement
- 20: Klammerelement
- 21: Schenkel
- 22: Spitze
- 23: Rücken
- 24: Aufnahmeelement
- 25: Schnappelement
- 26: Schnappnase
- 27: Drucktaste
- 28: Radiaxlager
- 29: Achsendbereich
- 30: Öffnung
- 31: Stift
- 32: Führungselement
- 33: Langloch
- 34: Schmalseite
- 35: Längsseite
- 36: Rotationsachse
- 37: gerader Abschnitt
- 38: Ausbuchtung
- 39: Zunge
- 40: Hebel
- 41: Griffelement
- 42: Rastnase
- 43: Rastloch
- 44: Instrumentenaufnahme
- 45: Durchführungselement
- 46: Sterilisationsfolie

## Patentansprüche

1. Trokarhalterung für einen Manipulator eines robotischen Operationssystems, umfassend
- ein Basiselement (19) zur Befestigung der Trokarhalterung am Manipulator
- ein Klammerelement (20), welches über einen Kopplungsmechanismus mit dem Basiselement (19) auswechselbar verbunden ist und an welchem zwei relativ zueinander in einer Klammerebene bewegliche, über einen Rücken (23) verbundene Schenkel (21) mit freien Spitzen (22) ausgebildet sind,
- ein Aufnahmeelement (24) für einen Trokar (18) und
- einen Klammermechanismus zum Öffnen und Schließen der Schenkel (21), **dadurch gekennzeichnet, dass**
- im Bereich der Spitzen (22) jedes der beiden Schenkel (21) ein Radiaxlager (28) ausgebildet ist,
- das Aufnahmeelement (24) als Achse ausgebildet ist und eine durchgehende Öffnung (30) zur Durchführung des Trokars (18) senkrecht durch die Achse aufweist,
- wobei das Aufnahmeelement (24) bei geschlossenen Schenkeln (21) von den Radiaxlagern (28) an einer axialen Bewegung gehindert ist.

2. Trokarhalterung nach Anspruch 1, **dadurch gekennzeichnet, dass** das Aufnahmeelement (24) bei geschlossenen Schenkeln (21) von diesen um eine in der Klammerebene liegende erste Rotationsachse rotierbar gehalten wird.

3. Trokarhalterung nach einem der Ansprüche 1 oder 2, **dadurch gekennzeichnet, dass** der Kopplungsmechanismus als mit der Hand ohne weitere Hilfsmittel bedienbarer Schnellverschluss, bevorzugt als Schnappverbindung ausgebildet ist.

4. Trokarhalterung nach einem der Ansprüche 1 bis 3, **dadurch gekennzeichnet, dass**
- mindestens das Klammerelement, das Basiselement und das Aufnahmeelement aus Kunststoff gefertigt sind und/oder
- die Schenkel (21) und der Rücken (23) des Klammerelements (20) einstückig gefertigt sind und / oder
- mindestens einer der Schenkel (21) federnd mit dem Rücken (23) verbunden ist.

5. Trokarhalterung nach einem der Ansprüche 1 bis 4, **dadurch gekennzeichnet, dass** zwischen dem Basiselement (19) und dem Klammerelement (20) eine Sterilisationsfolie (46) fixiert ist, wobei mindestens das Klammerelement (20), das Aufnahmeelement (24) und der Klammermechanismus steril ausgelegt sind.

6. Trokarhalterung nach einem der Ansprüche 1 bis 5, **dadurch gekennzeichnet, dass** das Aufnahmeelement (24) fest mit einem Trokar (18) verbunden ist.

## Claims

1. A trocar holder for a manipulator of a robotic operating system, comprising
- a base element (19) for attaching the trocar holder to the manipulator,
- a clamping element (20) which is interchangeably connected to the base element (19) via a coupling mechanism and on which two legs (21) with free tips (22) are formed which are movable relative to one another in a clamping plane and are connected via a back (23),
- a receiving element (24) for a trocar (18) and
- a clamping mechanism for opening and closing the legs (21), **characterised in that**
- a radiax bearing (28) is formed in the area of the tips (22) of each of the two legs (21),
- the receiving element (24) is designed as an axle and has a through hole (30) for the trocar (18) to pass perpendicularly through the axle,
- wherein an axial movement of the receiving element (24) is prevented by the radiax bearings (28) when the legs (21) are closed.

2. The trocar holder according to claim 1, **characterised in that**, when the legs (21) are closed, the receiving element (24) is held by them so as to be rotatable about a first axis of rotation lying in the clamping plane.

3. The trocar holder according to any one of claims 1 or 2, **characterised in that** the coupling mechanism is designed as a quick-release fastener which can be operated by hand without further aids, preferably as a snap connection.

4. The trocar holder according to any one of claims 1 to 3, **characterised in that**
- at least the clamping element, the base element and the receiving element are made of a plastic material and/or
- the legs (21) and the back (23) of the clamping element (20) are made in one piece and/or
- at least one of the legs (21) is resiliently connected to the back (23).

5. The trocar holder according to any one of claims 1 to 4, **characterised in that** a sterilisation foil (46) is fixed between the base element (19) and the clamping element (20), wherein at least the clamping element (20), the receiving element (24) and the clamp mechanism are designed to be sterile.

6. The trocar holder according to any one of claims 1 to 5, **characterised in that** the receiving element (24) is firmly connected to a trocar (18).

## Revendications

1. Support de trocart pour un manipulateur d'un système d'exploitation robotisé, comprenant
- un élément de base (19) pour fixer le support de trocart au manipulateur,
- un élément de serrage (20), qui est relié de manière interchangeable à l'élément de base (19) par un mécanisme d'accouplement et sur lequel deux jambes (21), qui sont mobiles l'une par rapport à l'autre dans un plan de serrage et qui sont reliées par un dos (23), sont formées avec des pointes libres (22),
- un élément récepteur (24) pour un trocart (18) et
- un mécanisme de serrage pour ouvrir et fermer les jambes (21), **caractérisé en ce que**
- dans la région des pointes (22) de chacune des deux jambes (21), un palier radial et de butée (28) est formé,
- l'élément récepteur (24) est réalisé sous forme d'un axe et comporte une ouverture de passage (30) pour le passage perpendiculaire du trocart (18) à travers l'axe,
- dans lequel l'élément récepteur (24) est empêché de se déplacer axialement par les paliers radiaux et de butée (28) lorsque les jambes (21) sont fermées.

2. Support de trocart selon la revendication 1, **caractérisé en ce que** l'élément récepteur (24), lorsque les jambes (21) sont fermées, est maintenu par ces dernières de manière à pouvoir tourner autour d'un premier axe de rotation situé dans le plan de serrage.

3. Support de trocart selon l'une quelconque des revendications 1 ou 2, **caractérisé en ce que** le mécanisme d'accouplement est réalisé sous forme d'une attache rapide qui peut être actionnée à la main sans autres moyens auxiliaires, de préférence sous forme d'une liaison à déclic.

4. Support de trocart selon l'une quelconque des revendications 1 à 3, **caractérisé en ce que**
- au moins l'élément de serrage, l'élément de base et l'élément récepteur sont en matière plastique et/ou
- les jambes (21) et le dos (23) de l'élément de serrage (20) sont fabriqués d'un seul tenant et/ou
- au moins une des jambes (21) est élastiquement reliée au dos (23).

5. Support de trocart selon l'une quelconque des revendications 1 à 4, **caractérisé en ce qu'**une feuille de stérilisation (46) est fixée entre l'élément de base (19) et l'élément de serrage (20), au moins l'élément de serrage (20), l'élément récepteur (24) et le mécanisme de serrage étant conçus pour être stériles.

6. Support de trocart selon l'une quelconque des revendications 1 à 5, **caractérisé en ce que** l'élément récepteur (24) est fermement relié à un trocart (18).
